(19) Europäisches Patentamt
European Patent Office
Office européen des brevets

(11) Publication number: 0 229 313 B1

(12) **EUROPEAN PATENT SPECIFICATION**

(45) Date of publication of the patent specification: **07.11.90**

(51) Int. Cl.[5]: **C07C 257/04,** C07C 233/47, C07C 233/49, C07C 271/22

(21) Application number: **86117114.8**

(22) Date of filing: **09.12.86**

(54) **Compound related to antibiotic TAN-749 and their production.**

(30) Priority: **27.12.85 JP 298671/85**

(43) Date of publication of application: **22.07.87 Bulletin 87/30**

(45) Publication of the grant of the patent: **07.11.90 Bulletin 90/45**

(84) Designated Contracting States: **AT BE CH DE ES FR GB IT LI LU NL SE**

(56) References cited:
**THE JOURNAL OF ANTIBIOTICS, vol. 29, no. 9, September 1976, pages 937-943, Tokyo, JP; Y. UEHARA et al.: "Structure-activity relationships among negamycin analogs"**

(73) Proprietor: **Takeda Chemical Industries, Ltd., 27, Doshomachi 2-chome Higashi-ku, Osaka-shi Osaka-fu(JP)**

(72) Inventor: **Harada, Setsuo, 3-31 Seiwadainishi 2-chome, Kawanishi Hyogo 666-01(JP)**
Inventor: **Ono, Hideo, 12-7, Uzumoridai 4-chome Higashinada-ku, Kobe Hyogo 658(JP)**

(74) Representative: **von Kreisler, Alek, Dipl.-Chem. et al, Deichmannhaus am Hauptbahnhof, D-5000 Köln 1(DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

EP 0 229 313 B1

## Description

The present invention relates to compounds related to a novel antibiotic TAN-749 (hereinafter abbreviated to "TAN-749" in some cases), which can be used favorably as a therapeutic drug for bacterial infectious diseases, and to the methods of their production.

δ-hydroxy-β-lysine, an amino acid for TAN-749A and C (described later), is known to be an amino acid as the constituent of the antibiotic negamycin [Refer to the Journal of the American Chemical Society, 93, 6305 (1971)].

Due to the development of therapeutics based on antibiotics, bacterial diseases have, for the most part, been overcome; however, there are still some important problems in the field of infectious disease medicine. For example, long-term or high-dose medication with conventional antibiotics causes changes in the flora of disease-causative bacteria (replacement of bacteria) or the advent of drug-resistant bacteria (acquisition of drug-resistance), resulting in an increase in diseases. Antibiotics possessing a novel structure and thus novel biological activity, or intermediate materials for their synthesis, are needed to solve these problems.

The present inventors isolated a great number of bacterial species from the soil in their search for new antibiotics and then separated and investigated antibiotics produced by those species, finding that some microbes belonging to the genus Pseudomonas produced a new antibiotic, which possesses antibacterial activity on both gram-positive and gram-negative bacteria including drug-resistant strains, and can be accumulated in a medium by culturing the relevant microbes in the medium. The inventors then separated this antibiotic and on the basis of its physico-chemical and biological properties, proved that it was a new antibiotic; it was named antibiotic TAN-749. TAN-749 is consisted of 4 components; they were named TAN-749A, B, C and D. As the results of further investigation, each TAN-749 component was found to be expressed by the following structures:

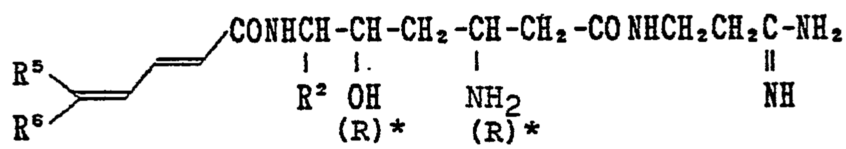

| No. | TAN-749 | $R^5$ | $R^6$ | $R^2$ |
|---|---|---|---|---|
| 1 | A | $-CH_3$ | -H | -H |
| 2 | B | $-CH_3$ | -H | $-CH_3$ |
| 3 | C | -H | $-CH_3$ | -H |
| 4 | D | -H | $-CH_3$ | $-CH_3$ |

* (R) means a R-configuration according to R-S rotation.

The inventors further carried out decomposition reaction using TAN-749 as the starting material to obtain an intermediate material to be used to synthesize its derivatives possessing higher biological activity.

Based on these findings, the inventors made further studies developing the present invention.

The present invention relates to:

(I) A compound representable by the formula

$$R^1-NH-\underset{R^2}{CH}-\underset{OH}{CH}-CH_2-\underset{R^3}{CH}-CH_2-COR^4 \quad (I)$$

wherein $R^1$ is hydrogen, hexanoyl, or sorbyl, $R^2$ is hydrogen or methyl, $R^3$ is amino which may optionally be protected, and $R^4$ is hydroxyl or 2-amidino-ethylamino, with the proviso that when both $R^1$ and $R^2$ are

hydrogen and $R^3$ is amino, $R^4$ is 2-amidino-ethylamino, or when $R^1$ is sorbyl and $R^4$ is 2-amidino-ethylamino, $R^3$ is a protected amino, or salts thereof.

(2) A method for preparing a compound representable by the formula

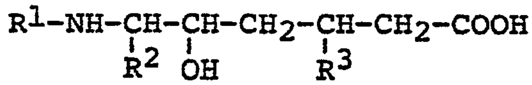

(II)

wherein $R^1$, $R^2$ and $R^3$ are the same meaning as defined above, with the proviso that when both $R^1$ and $R^2$ are hydrogen, $R^3$ is a protected amino, or salts thereof, which comprises subjecting a compound representable by the formula

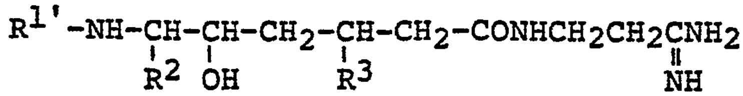

(III)

wherein $R^{1'}$ is hexanoyl or sorbyl, $R^2$ and $R^3$ are the same meaning as defined above, or salts thereof, to hydrolysis and

(3) A method for preparing a compound represnetable by the formula

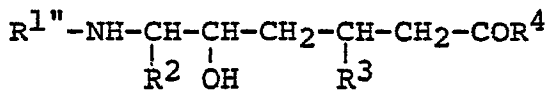

(IV)

wherein $R^{1''}$ is hydrogen or hexanoyl, $R^2$, $R^3$ and $R^4$ are the same meaning as defined above with the proviso that when both $R^{1''}$ and $R^2$ are hydrogen and $R^3$ is amino, $R^4$ is 2-amidino-ethylamino, or salts thereof, which comprises subjecting a compound representable by the formula

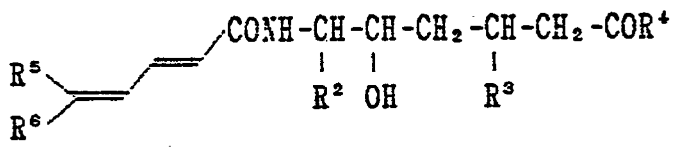

(V)

wherein $R^2$, $R^3$ and $R^4$ are the same meaning as defined above and $R^5$ and $R^6$ each is hydrogen or methyl, with the proviso that $R^5$ and $R^6$ are not simultaneously hydrogen or methyl, or salts thereof, to catalytic reduction, and when necessary, further to deacylation.

Protective groups for the amino group represented by $R^3$ in these formulas include aromatic acyl groups such as phthaloyl, benzoyl, p-nitrobenzoyl, p-tert-butylbenzoyl, p-tert-butylbenzenesulfonyl, benzenesulfonyl and toluenesulfonyl; aliphatic acyl groups such as formyl, acetyl, propionyl, monochloroacetyl, dichloroacetyl, trichloroacetyl, methanesulfonyl, ethanesulfonyl, trifluoroacetyl, maleyl and succinyl; esterified carbonyl groups such as methoxycarbonyl, ethoxycarbonyl, t-butoxycarbonyl, isopropoxycarbonyl, 2-cyanoethoxycarbonyl, β,β,β-trichloroethoxycarbonyl, benzyloxycarbonyl, p-nitrobenzyloxycarbonyl, p-methoxybenzyloxycarbonyl, diphenylmethyloxycarbonyl, methoxymethyloxycarbonyl, acetylmethyloxycarbonyl and phenyloxycarbonyl; methylene groups such as (hexahydro-lH-azepin-l-yl)methylene; sulfonyl groups such as 2-amino-2-carboxyethylsulfonyl; and other groups other than acyl groups, such as trityl, 2-nitrophenylthio, benzylidene, 4-nitrobenzylidene, di- or trialkylsilyl, benzyl and p-nitrobenzyl. Although there are no special limitations in choosing the above mentioned protective groups, it is particularly preferable that p-nitrobenzoyl, acetyl, t-butoxycarbonyl, benzyloxycarbonyl, etc. is used.

Compounds having a hydroxyl group for $R^4$ in the previous formulas sometimes form a lactone ring as follows:

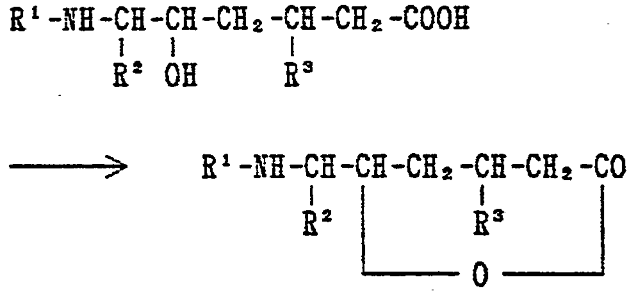

Compounds having a lactone ring as shown above are involved in the present invention.

The methods for preparing compounds involved in the present invention are hereinafter described.

The conventional acid hydrolysis method is used to eliminate the 2-amidino-ethylamino group from Compound (III) or its salts. That is, Compound (III) is dissolved in 2N hydrochloric acid to a concentration of 5 to 20 mg/ml and then refluxed for 5 minutes to 1 hour, preferably 15 to 40 minutes, while being heated (outside temperature: approx. 120°C)(Method I).

After the neutralization of the reaction liquid, the reaction product is purified by column chromatography using Diaion HP-20 (Mitsubishi Kasei)etc. as the packing. When the reflux is continued for 2 to 10 hours, preferably 4 to 8 hours, under the same conditions, the sorbyl or hexanoyl group is also eliminated from Compound(III), yielding a compound having no groups of 2-amidino-ethylamino, sorbyl and hexanoyl (Method II). The reaction product is purified by column chromatography using Dowex 50W (Dow Chemical, USA) etc. as the column

When Compound (V) or its salt is subjected to catalytic reduction, a compound having a sorbyl group whose 2 double bonds are saturated is obtained. This catalytic reduction is carried out using conventional reactions. That is, Compound (V) is dissolved in a polar solvent such as water or acetic acid; a catalyst for catalytic reduction such as platinum oxide, palladium-carbon, or Raney nickel is added, after which the solution is stirred in a hydrogen gas flow. The reaction take several hours to complete at normal temperature under normal pressure; it can be carried out under increased pressure to decrease reaction time.

It is recommended that both Compound (V) and compounds obtained by either the acid hydrolysis method (Method I) or the catalytic reduction method is treated so that their amino groups are protected by a protective group before being used for the next reaction. A common method of introducing a protective group to an amino group is described in detail by T. W. Greene in "Protective Groups in Organic Synthesis", p.218 (1981), John Wiley & Sons. Typical cases of the introduction of an N-protective group are as follows: In the case of t-butoxycarbonylation, the sample is dissolved in a polar solvent such as a mixture of 50% dioxane and water and approx. 1 ∿ 4 equivalent triethylamine and approx. 1 ∿ 3 equivalent 2-(t-butoxycarbonyloxyimino)-2-phenylacetonitrile (hereinafter abbreviated to BOC-ON in some cases) are added; the reaction is carried out by stirring the solution at normal temperature for 0.5 to 8 hours, preferably 3 to 6 hours. In the case of either benzoylation or benzyloxycarbonylation, the sample is dissolved in dilute sodium bicarbonate water and approx. 1 ∿ 3 equivalent benzoyl chloride or benzyloxycarbonyl chloride is added; the reaction is carried out at normal temperature for some 2 to 8 hours while stirring the solution.

Either Compound (VI) representable by the formula(V) wherein $R^3$ is amino group and $R^4$ is 2-amidino-ethylamino, or the catalytic reduction product from the acid hydrolysate (by Method I) described above, is treated so that the fatty acid group is eliminated either as it is or after the introduction of an N-protective group. The elimination is carried out using an enzyme, i.e. deacylase. Deacylases which can be used include deacylase contained in the bacterial cells of Pseudomonasacidovorans IFO 13582. When the said deacylase is used for the reaction, the bacterial cells are supplied either as they are or in the form of crude powder, previously treated with acetone etc. The sample is dissolved in a buffer solution such as a phosphoric acid or acetic acid buffer solution (pH: 5 to 9, preferably 6 to 7.5; ion concentration: 0.01 to 0.3M, preferably 0.02 to 0.2M), after which either the bacterial cells or the crude powder is added so that its concentration to 1 mg of the sample is 5 to 500 mg/mℓ, preferably 20 to 100 mg/mℓ. Reaction temperature is 30 to 45°C, preferably 34 to 38°C; reaction time is l0 to 48 hours, preferably l5 to 24 hours. This reaction is generally carried out under aeration while stirring. The reaction liquid is then subjected to centrifugation etc. to remove bacterial cells, after which it is purified by the ion exchange resin method etc.

Compound (III) produces Compound (II) via alkali hydrolysis. This reaction goes in two steps. That is, antibiotic TAN-749, catalytic reduction products from it, or their N-protective group introduction products are first treated under mild conditions so that the 2-amidino-ethylamide group is eliminated. The treating conditions are as follows: for example, Compound (III) is dissolved in a solution of sodium hydroxide; the solution is stirred. Sodium hydroxide concentration is 0.5 to 2 normal, preferbly l to l.5 normal. Reaction time is l day to 2 weeks, preferably 2 to 8 days when reaction temperature is 20 to 40°C, or

1 to 15 hours, preferably 2 to 8 hours when reaction temperature is 50 to 70°C, preferably 55 to 65°C (Method III). When more severe reaction conditions are used, not only the 2-amidino-ethylamino group but also either the sorbyl group or the hexanoyl group are eliminated from Compound (III), yielding a compound having neither 2-amidino-ethylamino group, nor sorbyl group, nor hexanoyl group (Compound II having a hydrogen atom for $R^1$). In this case, the reaction is carried out by maintaining the same alkaline reaction liquid as that described above at a reaction temperature of 50 to 70°C, preferably 55 to 65°C for 8 hours to 3 days, preferably 10 to 24 hours (Method IV).

Tables 1 and 2 show the main reaction processes described above and the structural formulas of various compounds obtained via those processes, respectively.

Table 1

| Reaction Process | Starting Material | Reaction Product |
|---|---|---|
| 1) Acid hydrolysis (Method I) | 1 | 15 |
| | 2 | 16 |
| | 8 | 18 |
| | 9 | 19 |
| 2) Acid hydrolysis (Method II) | 1 | 23 |
| | 16 | 24 |
| 3) Catalytic reduction | 1 or 3 | 8 |
| | 2 or 4 | 9 |
| | 15 | 18 |
| | 16 | 19 |
| | 17 | 20 |
| 4) Deacylation | 8 | 12 |
| | 10 | 13 |
| | 11 | 14 |
| | 18 | 23 |
| | 19 | 24 |
| | 20 | 25 |
| | 21 | 26 |
| 5) Introduction of N-protective group | 1 | 5 |
| | 1 | 6 |
| | 1 | 7 |
| | 8 | 10 |
| | 9 | 11 |
| | 15 | 17 |
| | 18 | 20 |
| | 18 | 22 |
| | 19 | 21 |
| 6) Alkali hydrolysis (Method III) | 6 | 17 |
| | 10 | 20 |
| | 11 | 21 |
| 7) Alkali hydrolysis (Method IV) | 10 | 25 |
| | 21 | 26 |

Table 2

$$R^1\text{-NH-}\underset{R^2}{\underset{|}{CH}}\text{-}\underset{OH}{\underset{|}{CH}}\text{-}CH_2\text{-}\underset{R^3}{\underset{|}{CH}}\text{-}CH_2\text{-}COR^4$$

| Compound | $R^1$ | $R^2$ | $R^3$ | $R^4$ |
|---|---|---|---|---|
| 5 | $CH_3CH=CH-CH=CH-CO-$ | H- | $C_6H_5CONH-$ | $-NHCH_2CH_2C(=NH)NH_2$ |
| 6 | 〃 | 〃 | $CH_3C(CH_3)_2OCONH-$ | 〃 |
| 7 | 〃 | 〃 | $C_6H_5CH_2OCONH-$ | 〃 |
| 8 | $CH_3(CH_2)_4CO-$ | H- | $-NH_2$ | 〃 |
| 9 | 〃 | $CH_3-$ | 〃 | 〃 |
| 10 | 〃 | H- | $CH_3C(CH_3)_2OCONH-$ | 〃 |
| 11 | 〃 | $CH_3-$ | 〃 | 〃 |
| 12 | H- | H- | $-NH_2$ | 〃 |
| 13 | 〃 | 〃 | $CH_3C(CH_3)_2OCONH-$ | 〃 |
| 14 | 〃 | $CH_3-$ | 〃 | 〃 |
| 15 | $CH_3CH=CH-CH=CH-CO-$ | H- | $-NH_2$ | -OH |
| 16 | 〃 | $CH_3-$ | 〃 | 〃 |
| 17 | 〃 | H- | $CH_3C(CH_3)_2OCONH-$ | 〃 |
| 18 | $CH_3(CH_2)_4CO-$ | H- | $-NH_2$ | 〃 |
| 19 | 〃 | $CH_3-$ | 〃 | 〃 |
| 20 | 〃 | H- | $CH_3C(CH_3)_2OCONH-$ | 〃 |
| 21 | 〃 | $CH_3-$ | 〃 | 〃 |
| 22 | 〃 | H- | $C_6H_5CH_2OCONH-$ | 〃 |
| 23 | H- | H- | $-NH_2$ | -OH |
| 24 | 〃 | $CH_3-$ | 〃 | 〃 |
| 25 | 〃 | H- | $CH_3C(CH_3)_2OCONH-$ | 〃 |
| 26 | 〃 | $CH_3-$ | 〃 | 〃 |

. TAN-749 to be used as the starting material for the present invention can be produced by the methods described later in Reference Example.

The strain Pseudomonasfluorescens YK-437, used in Reference Examples I and 2, has been deposited under the accession number of IFO 14446 at the Institute for Fermentation, Osaka (IFO) since June 7, I985. This microorganism, which was deposited on June I5, 1985 at the Fermentation Research Institute, Agency of Industrial Science and Technology, Ministry of International Trade and Industry, Japan (FRI) under the accession number of FERM P-83I2, the deposit being converted to a deposit under the Budapest Treaty, has been stored at FRI under the accession number of FERM BP-I005.

Compound (I), obtained by the present invention, can be used as a raw material to synthesize TAN-749, an antibiotic which can be used favorably as a therapeutic drug for bacterial infectious diseases.

TAN-749 can be synthesized from Compound (I) using the following production method: For example, Compound (I) is either dissolved or suspended in dimethylformamide; 1 ∿ 2 equivalent triethylamine, 1 ∿ 2 equivalent 1-hydroxybenzotriazole, and 1 ∿ 2 equivalent dicyclohexylcarbodiimide are added while cooling the solution or suspension with ice. The mixture is then stirred for 2 ∿ 16 hours either at normal temperature or under ice cooling conditions to yield TAN-749 whose amino group is protected by a t-butoxycarbonyl group (hereinafter referred to as N-BOC body in some cases); the resulting N-BOC body

is treated with trifluoroacetic acid at normal temperature for 5 ∿ 30 minutes. The N-BOC body of TAN-749 can also be obtained by dissolving Compound (I) in a dilute alkali solution, adding sorbyl chloride, and then stirring the solution at normal temperature for 30 minutes to 2 hours.

The biological characteristics of TAN-749 obtained from Compound (I) involved in the present invention are described hereinafter.

Tables 3 and 4 show the antibacterial spectra of TAN-749A, B, C, and D (dihydrochlorides) against various microbes.

Table 3

| Test Organism | Minimal Inhibitory Concentration (Note 1) (μg/mℓ) A | B | C | D |
|---|---|---|---|---|
| *Staphylococcus aureus* FDA 209P | 50 | 12.5 | >100 | 50 |
| *Escherichia coli* NIHJ JC2 | >100 | >100 | >100 | >100 |
| *Citrobacter freundii* IFO 12681 | ≧100 | ≧100 | >100 | >100 |
| *Klebsiella pneumoniae* IFO 3317 | >100 | 100 | >100 | >100 |
| *Proteus vulgaris* IFO 3988 | 100 | 25 | 100 | 100 |
| *Proteus morganii* IFO 3168 | >100 | 100 | >100 | >100 |
| *Pseudomonas aeruginosa* IFO 3080 | 25 | 50 | 50 | 100 |
| *Alcaligenes faecalis* IFO 13111 | 3.13 | 6.25 | 12.5 | 6.25 |
| *Acinetobacter calcoaceticus* IFO 13006 | 25 | 50 | >100 | >100 |

(Note 1) Medium composition

| | |
|---|---|
| Bacto-Antibiotic Medium 3 (Difco Laboratories, USA) | : 17.5g |
| Bacto-yeast extract (Difco Laboratories, USA) | : 5.0g |
| Bacto-agar (Difco Laboratories, USA) | : 20g |
| Distilled water (pH unadjusted) | : 1,000mℓ |
| Inoculum size | : Approx. $10^6$ CFU/mℓ |

Table 4

| Test Organism | Medium (Note 2) | Minimal Inhibitory Concentration (Note 1) (μg/mℓ) A | B | C | D |
|---|---|---|---|---|---|
| Staphylococcus aureus 308A-1 | TSA | 12.5 | 3.13 | >100 | 25 |
| Escherichia coli T7 | TSA | 50 | 12.5 | >100 | 50 |
| Staphylococcus aureus FDA209P | B-TSA | 12.5 | 3.13 | >100 | 25 |
| Streptococcus pyogenes E-14 | B-TSA | 3.13 | 6.25 | 100 | 25 |
| Pseudomonas aeruginosa P9 | B-TSA | 100 | 50 | 100 | 100 |

(Note 1) Determined by the agar dilution method. Inoculum size was $10^8$ CFU/mℓ
(Note 2) TSA (Tripticase Soy Agar; Baltimore Biological Laboratories, USA), B-TSA; 10% horse serum/TSA

Table 5 shows the antibactericidal activities of TAN-749A dihydrochloride against clinically isolated Staphylococcusaureus strains.

Table 5

| Strain | Resistance Type | Minimal Inhibitory Concentration (ug/mℓ) (Note 1) |
|---|---|---|
| 1840 S | None | 12.5 |
| 1840-2 | Penicillin G | 12.5 |
| TN 2613 | Methicillin | 6.25 |
| TN 2648 | Methicillin | 3.13 |
| TN 2687 | Macrolide. | 6.25 |
| TN 2684 | Macrolide | 6.25 |
| TN 2688 | Macrolide | 6.25 |

(Note 1) Determined by the agar dilution method.
Media: Mueller Hinton medium (Difco Laboratories, USA)
Inoculum size: $10^6$CFU/mℓ

Table 6 shows the therapeutic effects of TAN-749A, B, C, and D (dihydrochlorides) on infectious diseases in mice.

Table 6

| Infectious Bacterium | Route | $ED_{50}$ (mg/kg) A | B | C | D |
|---|---|---|---|---|---|
| *Escherichia coli* 0-111 | Subcutaneous | 67.2 | 27.3 | 50 | |
| *Escherichia coli* T7 | Subcutaneous | 50.8 | | | |
| *Pseudomonas aeruginosa* P-9 | Subcutaneous | 31.0 | 61.4 | | |
| *Staphylococcus aureus* 308A-1 | Subcutaneous | 1.31 | 0.351 | 12.5 | <6.25 |
| *Staphylococcus aureus* 308A-1 | Oral | 17.7 | 16.2 | | |

Table 7 shows the acute toxicities of TAN-749A and B (dihydrochlorides) on mice.

Table 7

| Route | $LD_{50}$ (mg/kg) A | B |
|---|---|---|
| Subcutaneous | Approx. 500 | 400∿800 |
| Oral | 2,000∿4,000 | |

As is clear from these data, TAN-749 and its salts possess antibacterial activity on both gram-positive and gram-negative bacteria and their toxicity on mammals is low. Moreover, they are effective on various drug-resistant bacteria and are not susceptible to cross-resistance. For these reasons, TAN-749 and its salts can be used in the therapeutics of bacterial infectious diseases in mammals (mice, rats, rabbits, dogs, humans, etc.).

TAN-749 or its salts can be used as therapeutic drugs for bacterial infectious diseases in the following ways. For example, TAN-749 or its salts, after mixing with pharmacologically allowable carriers, excipients, diluents, etc., is given non-orally to the said mammals via subcutaneous or intramuscular injection at a dose of approx. 1 to 50mg/kg/day, preferably approx. 5 to 20mg/kg/day. TAN-749 or its salts can be given orally in the form of capsules at a TAN-749 dose of approx. 1 to 100mg/kg/day; it is recommended that the dose be between approx. 5 and 50mg/kg/day.

TAN-749 or its salts can be used as bactericides. For example, hands, legs, eyes, ears, etc. can be sterilized and disinfected by applying to these areas a liquid prepared by dissolving TAN-749 or its salts in distilled water at a concentration of approx. 0.01 to 0.1w/v% or an ointment containing approx. 0.2 to 20mg, preferably approx. 1 to 10 mg, of TAN-749 per gram.

Compound (I) obtained by the present invention can work well as the intermediate material for the synthesis of TAN-749, an antibiotic which is useful as a therapeutic drug for bacterial infectious diseases.

The present invention is hereinafter described in more detail with reference examples and examples. Medium composition contents are expressed as weight.volume % unless otherwise stated.

YMC Packs S-30 and A3l2 (Yamamura Chemical Laboratories) were used as the preparative carrier and the analytical carrier for high performance liquid chromatography (hereinafter abbreviated HPLC in some cases), respectively.

Reference Example I

Pseudomonasfluorescens YK-437 (IFO I4446, FERM BP-1005) grown on an enriched agar slant medium was inoculated into a 2ℓ Sakaguchi flask containing 500mℓ of a medium prepared by adding 0.5% precipitating calcium carbonate to an aqueous solution (pH 7.0) containing 2% glucose, 3% soluble starch, 1% unprocessed soybean flour, 0.3% corn steep liquor, 0.5% Polypepton (Daigo Nutritive Chemical) and 0.3% sodium chloride, after which it was subjected to reciprocal shaking culture at 24°C for 48 hours. The entire quantity of the resulting culture liquid was then inoculated into a 50ℓ fermentor containing 30ℓ of a medium prepared by adding 0.05% Actcol (Takeda Chemical Industries), an antifoaming agent, to the said medium, and cultured at 24°C, with a 30ℓ/min. aeration rate and at 200 rpm for 48 hours. Six liters of the resulting culture liquid was then inoculated into a 200ℓ fermantor containing 120ℓ of a medium containing 3% glycerol, 0.1% glucose, 0.5% Polypepton (Daigo Nutritive Chemicals), 0.5% meat extract (Wako Pure Chemical Industries), 0.5% sodium chloride, 0.05% sodium thiosulfate, 2 ppm cobalt chloride and 0.05% Actcol, after which it was incubated at 24°C, with a 120ℓ/min. aeration rate and at 170 rpm for 66 hours.

The resulting culture liquid (105ℓ), after being adjusted to pH 6.5 with 2N hydrochloric acid, was added to a Hyflo super Cel (Jones Manville Product, USA) and subjected to filtration and water washing, yielding a filtrate (102ℓ). The resulting filtrate, after adjustment to pH 6.5, was passed through a column packed with IRC-50 ($Na^+$ type, 2ℓ). The column, after washing with water, was subjected to elution with a 2M saline solution (500ℓ). The resulting eluate was passed through a column packed with activated charcoal (2ℓ), washed with water, and then subjected to elution using an 8% isobutanol water solution (15ℓ) as an eluent. The resulting eluate, after adjusting to pH 6.2, was concentrated to 2ℓ and then passed through a column packed with CM-Sephadex C-25 ($Na^+$ type, 0.5ℓ). Active fractions were then eluted using a 0.1M saline solution (20ℓ) as an eluent.

A TAN-749B fraction appeared in the first half of the chromatogram and a TAN-749A fraction appeared in the last half of the chromatogram.

Each resulting fraction was subjected to chromatography using activated charcoal (1.0ℓ or 4.0ℓ) as the packing and then desalted, after which it was concentrated and lyophilized, yielding a crude TAN-749B product (4.0g) or a crude TAN-749A product (8.9g).

Crude product B (4.0g) was then subjected to reversed-phase high performance liquid chromatography for separation [Mobile phase: 8% methanol/0.02M phosphate solution (pH 3)], yielding an active fraction. The resulting active fraction was subjected to column chromatography using CM-Sephadex C-25 ($Na^+$ type, 0.25ℓ) and then subjected to column chromatography using activated charcoal (0.3ℓ), yielding a purified fraction. The resulting fraction was then concentrated and lyophilized, yielding TAN-749B dihydrochloride (0.66g) in the form of a white powder. Crude product A (8.9g) was treated with the same processes, yielding TAN-749A dihydrochloride in the form of a white powder (4.7g).

Reference Example 2

Pseudomonas fluorescens YK-437 (IFO I4446, FERM BP-1005) grown on an enriched agar slant meidum was inoculated into a 2ℓ Sakaguchi flask containing 500mℓ of a medium prepared by adding 0.5% precipitating calcium carbonate to an aqueous solution containing 2% glucose, 3% soluble starch, 1% unprocessed soybean powder, 0.3% corn steep liquor, 0.5% Polypepton, and 0.3% sodium chloride, after which it was subjected to reciprocal shaking culture at 24°C for 48 hours. The entire quantity of the resulting culture liquid was inoculated into a 200ℓ fermentor containing 120ℓ of a medium prepared by adding 0.05% Actcol, an antiforming agent, to the said medium, and then incubated at 24°C, with a 120ℓ/min. aeration rate and at 180 rpm for 48 hours. Fifty liters of the resulting culture liquid was inoculated into a 2,000ℓ fermentor containing 1,200ℓ of a medium containing 3% glycerol, 0.1% glucose, 0.5% Polypepton, 0.5% meat extract, 0.5% sodium chloride, 0.05% sodium thiosulfate, 2 ppm cobalt chloride and 0.05% Actcol, after which it was incubated at 24°C, with a 1,200ℓ/min. aeration rate and at 150 rpm for 66 hours.

The culture liquid obtained (1,150ℓ), after adjusting to pH 6.5, was added to a Hyflo super cell and the subjected to filtration and water washing, yielding a filtrate (1,220ℓ). The resulting filtrate, after adjustment to pH 6.2, was passed through a column packed with IRC-50 ($Na^+$ type, 20ℓ). The column, after washing with water, was subjected to elution using a 0.5M hydrochloric acid solution (200ℓ) as an eluent. The resulting eluate, after adjusting to pH 5.6, was passed through a column packed with Diaion SP-207 (20ℓ) and then subjected to elution with water (120ℓ). The resulting eluate was concentrated to 2ℓ the concentrate was passed through a column packed with CG-50 ($NH_4^+$ type, 3ℓ). Active fractions were then eluted using a 0.4 ∿ 0.6M saline solution (40ℓ) as an eluent.

TAN-749A, B, C and D fractions appeared in the first half of the chromatogram and a TAN-749A fraction appeared in the last half.

Each resulting fraction was subjected to chromatography using activated charcoal (1.2ℓ or 2.0ℓ) as the packing, and eluted with an 8% isobutanol water solution (4ℓ or 10ℓ). The fraction containing TAN-749A alone was concentrated and lyophilized, yielding TAN-749A (47.5g).

Three separate lots (corresponding to 3,450ℓ of the initial culture liquid) or the fraction containing TAN-749A, B, C and D, obtained by the same process, were concentrated in a lump, yielding a concentrate. The resulting concentrate (2ℓ) was then subjected to column chromatography using CG-50 ($NH_4^+$, 3ℓ) as the packing. The column, after washing with a 0.2M saline solution, was subjected to elution using a 0.5 ∿ 0.8M saline solution (40ℓ). A fraction containing TAN-749B and D appeared in the first half of the chromatogram and one containing TAN-749A and C appeared in the last half. The fraction containing TAN-749A and C was subjected to chromatography using activated charcoal as the packing and desalted. The resulting eluate was concentrated and lyophilized, yielding a TAN-749A powder (20g) containing a small quantity of TAN-749C.

The fraction containing TAN-749B and D was subjected to chromatography using activated charcoal as the packing and desalted. The resulting eluate was subjected to column chromatography using CM-Sephadex C-25 ($Na^+$ type, 1ℓ) as the packing and a 0.2M saline solution as an eluent. The resulting eluate was concentrated, and the resulting concentrate was subjected to reversed-phase HPLC for separation [Mobile phase: 5% methanol/0.02M phosphoric acid buffer solution (pH 3.0)], yielding two fractions, i.e. a fraction containing TAN-749B alone and one containing TAN-749B and D. The fraction containing TAN-749B alone was subjected to chromatography using CM-Sephadex and then activated charcoal as packings, yielding TAN-749B (3.05g). The fraction containing TAN-749B and D was concentrated and then subjected to HPLC again. The resulting fraction containing TAN-749D alone was then concentrated. The resulting concentrate was passed through a column packed with IRA-402 ($Cℓ^-$ type, 10mℓ) and the column was washed with water. The resulting eluate and the wash solution were subjected to chromatography using activated charcoal for desalting, yielding TAN-749D (15.5mg).

The TAN-749A powder (3g) containing a small quantity of TAN-749C, obtained above, was subjected to chromatography using CM-Sephadex, Amberlite CG-50 (Rohm & Hass Co., U.S.A.) and then activated charcoal as packings to increase the ratio of TAN-749C content. The resulting powder with a high TAN-749C content was purified via two repetitions of HPLC under the conditions shown above, yielding TAN-749C (20.2mg).

Referencke Example 3

The dihydrochloride of Compound 13 (964mg) was suspended in dimethylformamide (10mℓ). While cooling the suspension with ice, triethylamine (480μℓ), sorbic acid (307 mg), 1-hydroxybenzotriazole (369mg), and dicyclohexylcarbodiimide (563mg) were added and the resulting mixture was stirred for 1 hour while cooling with ice. The mixture was then returned to room temperature and stirred for 8 more hours. The reaction liquid was subjected to filtration, after which the solvent of the resulting filtrate was evaporated under reduced pressure and water (200mℓ) was added. The resulting solution, after adjusting to pH 2.5, was washed with ethyl acetate (100mℓ × 2). The water layer, after adjusting to pH 5.5, was concentrated and subjected to column chromatography using Diaion HP-20 (50 ∿ 100 mesh, 50mℓ) as the packing. The concentrate, after washing with water (200mℓ), was subjected to fractional elution using a series of 10% methanol-water (100mℓ), 50% methanol-water (100mℓ) and 50% methanol-1/200 N hydrochloric acid (200mℓ) as eluents. Each resulting fraction was subjected to high performance liquid chromatography [Mobile phase: 50% methanol/0.01M phosphoric acid solution (pH 3)]. The resulting fractions exhibiting a single peak were combined and concentrated, after which the resulting concentrate was lyophilized, yielding the hydrochloride of the t-butoxycarbonyl body of Compound 3 (999mg).

Optical rotation: $[\alpha]^{25}_D$ - 27.4° (c = 0.50, water)
Elemental analysis ($C_{20}H_{35}N_5O_5 \cdot HCℓ \cdot 0.5H_2O$)
Calculated: C; 51.00, H; 7.92, N; 14.87, Cℓ; 7.53 (%)
Found : C; 50.83, H; 8.01, N; 14.71, Cℓ; 7.57 (%)

The hydrochloride of the t-butoxycarbonyl body of Compound 3 (853mg) was dissolved in trifluoroacetic acid (5mℓ) and left in air at room temperature for 30 minutes. After evaporating the solvent, the reaction liquid was treated with ether, yielding a powdery substance (1,050mg). The powder was dissolved in water (70mℓ) and passed through a column packed with Amberlite IRA-402 (Cℓ type, Rohm & Hass Co., USA, 40mℓ). The column was then washed with water (40mℓ). The resulting eluate and the wash solution were combined and concentrated, after which the concentrate was lyophilized, yielding the dihydrochloride of Compound 3 (725mg). This compound was identical with the compound isolated from a natural source in their phisico-chemical properties.

Example I

The dihydrochloride of Compound 1 (approx. 200mg) was dissolved in water (20mℓ) and sodium bicarbonate (0.73g) and benzoyl chloride (0.175mℓ) were added, after which the solution was stirred at room temperature. With the disappearance of benzoyl chloride and pH reduction in the reaction liquid, both benzoyl chloride and triethylamine were added properly so that the reaction liquid was maintained at a pH value of approx. 8.3. Some 5 hours later, the reaction liquid was washed twice with ethyl acetate (35mℓ), adjusted to pH 2.0 with 2N HCℓ, and washed 3 times with ethyl acetate (30mℓ). The washed liquid, after adjusting to pH 6.7 with 3N sodium hydroxide, was concentrated and adsorbed to a column packed with Diaion HP-20 (50 ∿ 100 mesh, 20mℓ). The column was washed with water (120mℓ), after which the adsorbed concentrate was eluted with a 5% methanol water solution, a 20% methanol water solution, a 50% methanol water solution and 50% methanol-0.008N HCℓ (each 60mℓ) sequentially to fractionate 20mℓ portions of the eluate. Each resulting fraction was subjected to high performance liquid . chromatography [Mobile phase: 50% methanol/0.01M phosphoric acid solution (pH 3)]; the fractions exhibiting a single peak were combined together and concentrated, after which the resulting concentrate was lyophilized, yielding the hydrochloride of Compound 5 in the form of a white powder (167mg).

Optical rotation: $[\alpha]^{23}_D$ - 40.7° (c = 0.46, water)
Elemental analysis ($C_{22}H_{31}N_5O_4 \cdot HC\ell \cdot 1.0H_2O$)
Calculated: C; 54.60, H; 7.08, N; 14.47, Cℓ; 7.33 (%)
Found : C; 54.52, H; 6.92, N; 14.41, Cℓ; 7.66 (%)

Example 2

The dihydrochloride of Compound 1 (1.25g, 83% purity) was dissolved in 50% dioxane-water (50mℓ) and both triethylamine (0.5mℓ) and BOC-ON (1.1g) were added. The resulting mixture was stirred at room temperature for 5.5 hours while adding triethylamine to maintain pH of the mixture at approx. 8.5. The reaction liquid, after adjustment to pH 7.2 with 2N HCℓ, was concentrated to remove dioxane. Water (200mℓ) was added, after which the concentrate was washed with ethyl acetate-ethyl ether (1:1, 200mℓ). The organic layer was separated and further extracted with water (150mℓ). The resulting extract was combined with the water layer and concentrated. The resulting concentrate, after adjustment to pH 6.8, was passed through a column packed with Diaion HP-20 (50 ~ 100 mesh, 70mℓ). Elution was then carried out with water (210mℓ), 50% methanol-water (210mℓ) and 50% methanol-1/200 N HCℓ (280mℓ) sequentially to fractionate 70mℓ portions of the eluate. Each resulting fraction was analyzed by high performance liquid chromatography [Mobile phase: 50% methanol/0.01M phosphoric acid solution (pH 3)]. The fractions exhibiting a single peak were combined together and concentrated, after which the resulting concentrate was lyophilized, yielding the hydrochloride of Compound 6 (834 mg).

Optical rotation: $[\alpha]^{25}_D$ - 23.7° (c = 0.52, water)
Elemental analysis ($C_{20}H_{35}N_5O_5 \cdot HC\ell \cdot 1.5H_2O$)
Calculated: C; 49.12, H; 8.04, N; 14.32, Cℓ; 7.25 (%)
Found: C; 49.08, H; 8.07, N; 14.44, Cℓ; 7.26 (%)

Example 3

The dihydrochloride of Compound 1 (776mg) was dissolved in 3% sodium bicarbonate water solution (40mℓ) and carbobenzoxy chloride (798μℓ) was added, after which the solution was stirred at room temperature for 5 hours. The reaction liquid, after adjustment to pH 2, was diluted with water (50mℓ) and washed with ethyl acetate (100mℓ × 2). The water layer, after adjustment to pH 4.5, was concentrated and subjected to column chromatography using Diaion HP-20 (50 ~ 100 mesh, 40mℓ) as the packing. After washing with water (100mℓ) and then with 10% methanol-water (100mℓ), the concentrate was subjected to fractional elution using sequentially 50% methanol-water (100mℓ) and 50% methanol-1/200 N HCℓ (150mℓ). Each resulting fraction was analyzed by high performance liquid chromatography [Mobile phase: 60% methanol/0.01M phosphoric acid solution (pH 3)]. The fractions exhibiting a single peak were combined together and concentrated. The resulting concentrate was then lyophilized, yielding the hydrochloride of Compound 7 (728mg).

Optical rotation: $[\alpha]^{22}_D$ - 41.7° (c = 0.55, water)
Elemental analysis ($C_{23}H_{33}N_5O_5 \cdot HC\ell \cdot 0.5H_2O$)
Calculated: C; 54.70, H; 6.99, N; 13.87, Cℓ; 7.02 (%)
Found: C; 55.02, H; 6.85, N; 14.06, Cℓ; 7.29 (%)

Example 4

The dihydrochloride of Compound 1 (20.0g, approx. 97% purity) was dissolved in water (500mℓ) and 10% palladium-carbon (2.0g) was added, after which the solution was stirred at room temperature in a hydrogen gas flow for some 4 hours. The reaction liquid was subjected to filtration to remove the catalyst; the resulting filtrate was concentrated and lyophilized, yielding the dihydrochloride of Compound 8 in the form of a white powder (19.0g).
Optical rotation: $[\alpha]^{23.5}_D$ - 5.2° (c = 0.60, water)
Elemental analysis ($C_{15}H_{31}N_5O_3 \cdot 2HC\ell \cdot 1.0H_2O$)
Calculated: C; 42.86, H; 8.39, N; 16.66, Cℓ; 16.87 (%)
Found: C; 42.88, H; 8.84, N; 16.75, Cℓ; 17.26 (%)

Example 5

The dihydrochloride of Compound 2 (1.04g, 94% purity) was dissolved in water (100mℓ) and 10% palladium-carbon (104mg) was added, after which the solution was stirred at room temperature in a hydrogen gas flow for some 80 minutes. The reaction liquid was subjected to filtration to remove the catalyst; the resulting filtrate, after concentration, was passed through a column packed with activated charcoal (70mℓ). Sequential elution was then carried out using water (350mℓ) and then an 8% isobutanol water solution (500 mℓ) as eluents to fractionate 70mℓ portions of the eluate. Each resulting fraction was analyzed by high performance liquid chromatography [Mobile phase: 25% methanol/0.01M phosphoric acid solution (pH 3)]. The fractions exhibiting a single peak were combined together and concentrated, after which the resulting concentrate was lyophilized, yielding the dihydrochloride of Compound 9 in the form of a white powder (899mg).

Optical rotation: $[\alpha]^{24}_D + 28.9°$ (c = 0.57, water)
Elemental analysis ($C_{16}H_{33}N_5O_3{\cdot}2HC\ell{\cdot}0.5H_2O$)
Calculated: C; 45.18, H; 8.53, N; 16.46, Cℓ; 16.67 (%)
Found: C; 44.96, H; 8.82, N; 16.46, Cℓ; 17.10 (%)

Example 6

The dihydrochloride of Compound 8 (19.3g, 80% purity) was dissolved in 50% dioxane-water (400mℓ) and both triethylamine (7.65mℓ) and BOC-ON (13.5g) were added. The mixture was stirred at room temperature for 5 hours, while triethylamine was added to maintain a pH value of approx. 8.5. The reaction liquid, after adjustment to pH 6.5 with 2N HCℓ, was concentrated to remove dioxane. The resulting concentrate was diluted with water (900mℓ) and washed with ethyl acetate-ethyl ether (1:1, 800mℓ). The organic layer was separated and then further extracted with water (500mℓ). The resulting extract was combined with the water layer and concentrated, after which the resulting concentrate was adjusted to pH 5.6 and then passed through a column packed with Diaion HP-20 (50 ∿ 100 mesh, 450mℓ). Sequential elution was then carried out using a series of water (1.35ℓ), 50% methanol-water (1.35ℓ) and 50% methanol-1/200 N HCℓ (1.8ℓ) to fractionate 450mℓ portions of the eluate. Each resulting fraction was analyzed by high performance liquid chromatography [Mobile phase: 60% methanol/0.01M phosphoric acid solution (pH 3)]. The fractions exhibiting a single peak were combined together and concentrated, after which the resulting concentrate was lyophilized, yielding the hydrochloride of Compound 10 (13.4g).

Optical rotation: $[\alpha]^{25}_D - 13.3°$ (c = 0.67, water)
Elemental analysis ($C_{20}H_{39}N_5O_5{\cdot}2HC\ell$)
Calculated: C; 51.55, H; 8.65, N; 15.03, Cℓ; 7.61 (%)
Found : C; 51.22, H; 9.06, N; 14.82, Cℓ; 7.64 (%)

Example 7

The dihydrochloride of Compound 9 (700mg) was dissolved in a 50% dioxane water solution (28mℓ) and both triethylamine (0.28mℓ) and BOC-ON (455mg) were added. The mixture was then stirred at room temperature for some 8 hours while a pH value of approx. 8.8 was maintained using triethylamine. The reaction liquid was concentrated to remove dioxane. The resulting concentrate, after dilution with water (100mℓ), was washed twice with ether-hexane (5:1, 100mℓ). The organic layer was separated and extracted with water (150mℓ). The resulting extract was combined with the water layer; the mixture, after adjustment to pH 7, was concentrated and then adsorbed to Diaion HP-20 (50 ∿ 100 mesh, 30mℓ). Sequential elution was then carried out using a series of water, a 20% methanol water solution, a 50% methanol water solution and 50% methanol-0.005N dilute HCℓ water solution (each 120mℓ) to fractionate 20mℓ portions of the eluate. Each resulting fraction was analyzed by high performance liquid chromatography [Mobile phase: 60% methanol/0.01M phosphoric acid solution (pH 3)]. The fractions exhibiting a single peak were combined together and concentrated. The resulting concentrate was then lyophilized, yielding the hydrochloride of Compound 11 in the form of a white powder (487mg).

Optical rotation: $[\alpha]^{23}_D + 23.1°$ (c = 0.38, water)
Elemental analysis ($C_{21}H_{41}N_5O_5{\cdot}HC\ell{\cdot}H_2O$))
Calculated: C; 51.57, H; 8.86, N; 14.32, Cℓ; 7.25 (%)
Found : C; 51.40, H; 9.05, N; 14.21, Cℓ; 7.21 (%)

Example 8

The dihydrochloride of Compound 8 (202mg) was dissolved in a 0.03M phosphate buffer solution (pH 7.0, 100mℓ). After the addition of bacterial cells (10g) of <u>Pseudomonasacidovorans</u> IFO 13582, the solution was shaken at 37°C for 15 hours. The reaction liquid was centrifuged; the resulting supernatant, after adjustment to pH 7.0, was passed through a column packed with Amberlite CG-50 (100 ∿ 200 mesh, $H^+$ type, Rohm & Hass, USA, 40mℓ). After washing the column with a series of water (200mℓ) and 0.01N HCℓ (160mℓ), fractional elution was carried out using 0.02N HCℓ as the eluent. Each resulting fraction was analyzed by high performance liquid chromatography [Mobile phase: 30% acetonitrile/0.01M oc-

tanesulfonate-0.02M phosphoric acid solution (pH 3)]. The fractions whose main constituent was Compound 12, i.e. the desired product, were combined together and concentrated. The resulting concentrate was then lyophilized, yielding a crude powder (147mg). This crude powder was dissolved in a small amount of water and passed through a column packed with Diaion HP-20 (50 ∿ 100 mesh, 40mℓ). Fractional elution was then carried out. Each resulting fraction was analyzed by high performance liquid chromatography [Mobile phase: 30% acetonitrile/0.01M octanesulfonate-0.02M phosphoric acid solution )pH 3)]. The fractions exhibiting a single peak were combined together and concentrated, after which the resulting concentrate was lyophilized, yielding the trihydrochloride of Compound 12 (118mg).

Optical rotation: $[\alpha]^{25}_D$ - 7.6° (c = 0.45, water)
Elemental analysis ($C_9H_{21}N_5O_23HC\ell 2H_2O$)
Calculated: C; 28.70, H; 7.49, N; 18.59, Cℓ; 28.23 (%)
Found :C; 28.58, H; 7.19, N; 18.32, Cℓ; 28.41 (%)

Example 9

The hydrochloride of Compound 10 (10.0g) was dissolved in a 0.03M phosphate buffer solution (ph 7.0, 5.0ℓ). After the addition of bacterial cells (500g) of Pseudomonasacidovorance, the solution was shaken at 37°C for 15 hours. The reaction liquid was centrifuged; the resulting supernatant, after adjusting to pH 7.3, was passed through a column packed with IRC-50 ($Na^+$ type, 1ℓ). After washing the column with water (4ℓ), fractional elution was carried out using sequentially a 0.5M saline solution (8ℓ) and a 1.0M saline solution (5ℓ). Each resulting fraction was analyzed by high performance liquid chromatography [Mobile phase: 15% methanol/0.01M phosphoric acid solution (pH 3)]. The fractions exhibiting a single peak were combined together and passed through a column packed with charcoal powder (0.8ℓ). After washing the column with water (2ℓ), elution was carried out using a series of 8% isobutanol-water (4ℓ) and 8% isobutanol-1/100 N HCℓ (3ℓ). The eluate, after concentration, was lyophilized, yielding the dihydrochloride of Compound 13 (6.82g).

Optical rotation: $[\alpha]^{22}_D$ - 1.6° (c = 0.90, water)
Elemental analysis ($C_{14}H_{29}N_5O_42HC\ell 0.5H_2O$)
Calculated: C; 40.68, H; 7.80, N; 16.94, Cℓ; 17.15 (%)
Found :C; 40.62, H; 8.40, N; 17.04, Cℓ; 17.76 (%)

Example I0

The hydrochloride of Compound 11 (400mg) was dissolved in a 0.03M phosphate buffer solution (pH 7.0, 200mℓ). After adding bacterial cells (18g) of Pseudomonasacidovorans, the solution was shaken at 37°C for 25 hours. The reaction liquid was centrifuged and the resulting supernatant was passed through a column packed with IRC-50 ($NH_4^+$ type, 30mℓ). Elution was then carried out using water (100 mℓ), a 0.5M saline solution (150mℓ) and a 1.0M saline solution (100mℓ) sequentially to fractionate 20mℓ portions of the eluate. Each resulting fraction was analyzed by high performance liquid chromatography [Mobile phase: 15% methanol/0.01M phosphoric acid solution (pH 3)]. The fractions exhibiting a single peak were combined together and concentrated. The resulting concentrate was passed through a column packed with activated charcoal (20mℓ), after which it was eluted with water (100mℓ) and then with an 8% isobutanol water solution (100mℓ) to fractionate 20mℓ portions of the eluate. Each resulting fraction was analyzed by high performance liquid chromatography [Mobile phase: 15% methanol/0.01M phosphoric acid solution (pH 3)]. The fractions exhibiting a single peak were combined together and concentrated. The resulting concentrate was then lyophilized, yielding the dihydrochloride of Compound 14 in the form of a white powder (271mg).
Optical rotation: $[\alpha]^{21}_D$ + 4.0° (c = 0.55, water)
Elemental analysis ($C_{15}H_{31}N_5O_42HC\ell 0.5H_2O$)
Calculated: C; 42.16, H; 8.02, N; 16.39, Cℓ; 16.59 (%)
Found :C; 42.23, H; 8.61, N; 16.33, Cℓ; 16.78 (%)

Example II

The dihydrochloride of Compound 1 (50.2g, 83% purity) was dissolved in 2N hydrochloric acid (500mℓ) and refluxed in an oil bath for 15 minutes under heating at 130°C. The refluxed solution was concentrated to evaporate hydrochloric acid and diluted with water (60mℓ), after which it was adjusted to pH 6.8 with 1N aqueous sodium hydroxide and concentrated. The resulting concentrate was then passed

through a column packed with Diaion HP-20 (50 ∿ 100 mesh, 10ℓ) and eluted with water (3ℓ) and then with a 10% methanol water solution (5ℓ) to fractionate 1ℓ portions of the eluate. Each resulting fraction was analyzed by high performance liquid chromatography [Mobile phase: 20% methanol/0.0IM phosphoric acid solution (pH 3)], after which the fractions exhibiting a single peak were combined together, concentrated, and lyophilized, yielding Compound 15 in the form of a powder (1.79g).

Optical rotation: $[\alpha]^{21}_D$ - 22.3° (c = 0.52, water)
Elemental analysis ($C_{12}H_{20}N_2O_4 0.5H_2O$)
Calculated: C; 54.33, H; 7.98, N; 10.56 (%)
Found :C; 53.81, H; 8.11, N; 10.46 (%)

Example I2

The dihydrochloride of Compound 2 (1.25g, 86% purity) was dissolved in 2N hydrochloric acid (125mℓ) and refluxed in an oil bath for 18 hours under heating at 124°C. The reaction liquid was then cooled to room temperature and concentrated to evaporate hydrochloric acid. The resulting concentrate, after diluting with water, was adjusted to pH 6.8 with 1N aqueous sodium hydroxide. The diluted solution, after concentration, was passed through a column packed with Diaion HP-20 (50 ∿ 100 mesh, 150mℓ) and eluted sequentially with water (500mℓ), a 10% methanol water solution, a 20% methanol water solution and a 40% methanol water solution (each 450mℓ) to fractionate 150mℓ portions of the eluate. Each resulting fraction was analyzed by high performance liquid chromatography [Mobile phase: 25% methanol/0.0IM phosphoric acid solution (pH 3)]. The fractions exhibiting a single peak were combined together and concentrated, after which the resulting concentrate was lyophilized, yielding Compound 16 in the form of a white powder (449mg).
Optical rotation: $[\alpha]^{24}_D$ + 84.7° (c = 0.42, water)
Elemental analysis ($C_{13}H_{22}N_2O_4 1.0H_2O$)
Calculated: C; 54.15, H; 8.39, N; 9.72 (%)
Found :C; 54.55, H; 8.15, N; 9.72 (%)

Example I3

Compound 15 (3.4g) was dissolved in a 50% acetone water solution (114mℓ) and both triethylamine (7.35mℓ) and BOC-ON (3.92g) were added, after which the solution was stirred at room temperature for about 5 hours. After evaporation of acetone by concentration, the reaction liquid was adjusted to pH approx.8.8 by the addition of sodium bicarbonate (1.2g) and then washed with ethyl ether (100mℓ) 3 times. The washed solution, after adjusting to pH 2.0 with 1N HCℓ, was extracted 4 times with ethyl acetate (100mℓ). The resulting extracts were combined together, washed twice with a saline solution (100mℓ), dried with anhydrous sodium sulfate, and then concentrated to dryness, yielding a colorless, oily substance. The resulting substance was crystallized from an ethyl acetate-ethyl ether-hexane system, yielding Compound 17 in the form of white crystals (4.12g).

Melting point: 128.5°C
Optical rotation: $[\alpha]^{26}_D$ - 26.5° (c = 0.50, methanol)
Elemental analysis ($C_{13}H_{28}N_2O_6$)
Calculated: C; 57.29, H; 7.92, N; 7.86 (%)
Found :C; 57.34, H; 7.72, N; 7.98 (%)

Example I4

Compound 15 (600mg) was dissolved in water (60mℓ) and 10% palladium-carbon (60mg) was added, after which the solution was stirred at room temperature in a hydrogen gas flow for 3.5 hours. The reaction liquid was subjected to filtration, after which the resulting filtrate was adjusted to pH 7.0 and concentrated. The resulting concentrate was subjected to column chromatography using Diaion HP-20 (50 ∿ 100 mesh, 180mℓ) as the packing. After washing the column with water (720mℓ), fractional elution was carried out using 15% methanol-water (540mℓ) and then 25% methanol-water (540 mℓ) as eluents. Each resulting fraction was analyzed by high performance liquid chromatography [Mobile phase: 40% methanol/0.01M phosphoric acid solution (pH 3)]. The fractions exhibiting a single peak were combined together and concentrated, after which the resulting concentrate was lyophilized, yielding Compound 18 in the form of a powder (416mg).
Optical rotation: $[\alpha]^{22}_D$ - 13.9° (c = 0.51, water)
Elemental analysis ($C_{12}H_{24}N_2O_4$)
Calculated: C; 55.36, H; 9.29, N; 10.76 (%)
Found :C; 55.22, H; 9.20, N; 10.87 (%)

Example I5

Compound 17 (383mg) was dissolved in a solution of 0.1N NaOH (10.7mℓ) in water (30mℓ), and 10% palladium-carbon (40mg) was added. The mixture was stirred at room temperature in a hydrogen gas atmosphere for 5 hours. The reaction liquid was subjected to filtration, after which the resulting filtrate was adjusted to pH 7.0 and concentrated. The resulting concentrate was subjected to column chromatography [Mobile phase: 65% methanol/0.01M phosphoric acid solution (pH 3)] using Diaion HP-20 (50 ∿ 100 mesh, 30mℓ) as the packing. After washing the column with water (120mℓ), fractional elution was carried out using 10% methanol-water (60mℓ) and then 50% methanol-water (200mℓ). Each resulting fraction was analyzed by high performance liquid chromatography. The fractions exhibiting a single peak were combined together and concentrated, after which the resulting concentrate was lyophilized, yielding the sodium salt of Compound 20 (356mg).

Optical rotation: $[\alpha]^{23}_D$ - 7.7° (c = 0.48, water)
Elemental analysis ($C_{17}H_{31}N_2O_6Na$)
Calculated: C; 53.39, H; 8.17, N; 7.33 (%)
Found :C; 53.06, H; 8.01, N; 7.39 (%)

Example I6

Compound 16 (706mg) was dissolved in water (70mℓ) and 10% palladium-carbon (70mg) was added. The mixture was then stirred at room temperature in a hydrogen gas flow for about 1 hour. The reaction liquid was subjected to filtration to separate the catalyst. The resulting filtrate, after concentration, was crystallized from a water-acetone system, yielding Compound 19 in the form of white crystals (647mg).

Melting point: 204°C (decomposition)
Optical rotation: $[\alpha]^{21}_D$ + 31.8° (c = 0.57, water)
Elemental analysis ($C_{13}H_{26}N_2O_4$)
Calculated: C; 56.91, H; 9.55, N; 10.21 (%)
Found :C; 56.80, H; 9.82, N; 10.13 (%)

Example I7

Compound 18 (320mg) was dissolved in a 3% sodium bicarbonate water solution (25mℓ) and carbobenzoxy chloride (332μℓ) was added, after which the mixture was stirred at room temperature for 6 hours. The reaction liquid, after adjusting to pH 2, was extracted with ethyl acetate. After washing with water, the organic layer was dried with anhydrous sodium sulfate and then concentrated. Ethyl etherhexane was added to the concentrate, yielding Compound 22in the form of a powder (389mg).

Optical rotation: $[\alpha]^{22}_D$ - 0° (c = 0.49, methanol)
Elemental analysis ($C_{20}H_{30}N_2O_6$)
Calculated: C; 60.90, H; 7.67, N; 7.10 (%)
Found :C; 60.85, H; 7.53, N; 7.15 (%)

Example 18

Compound 19 (468mg) was dissolved in a 50% acetone water solution (16mℓ) and both triethylamine (0.95mℓ) and BOC-ON (504mg) were added, after which the mixture was stirred at room temperature for about 3 hours. After evaporating acetone and triethylamine by concentration, the reaction liquid was adjusted to pH 8.4 by adding sodium bicarbonate (160mℓ) and water (20mℓ). The diluted solution was washed 3 times with ethyl ether (30mℓ), after which it was adjusted to pH 2.2 with 2N HCℓ and then extracted 3 times with ethyl acetate (40mℓ). The resulting ethyl acetate layers were combined, washed twice with a saturated saline solution (20mℓ), desalted with anhydrous sodium sulfate, and then concentrated to dry, yielding a colorless, oily substance. This substance was then crystallized from an ether-hexane system, yielding Compound 21 in the form of white crystals (423mg).

Melting point: 102 ∿ 103°C Optical rotation: $[\alpha]^{23}_D$ + 36.0° (c = 0.45, methanol) Elemental analysis ($C_{18}H_{34}N_2O_6$)
Calculated: C; 57.73, H; 9.15, N; 7.48 (%)
Found :C; 57.81, H; 9.21, N; 7.47 (%)

Example I9

The dihydrochloride of Compound 1 (1.94g) was dissolved in 2N HCℓ (193mℓ) and refluxed for 6 hours under heating. After cooling, the reaction liquid was washed 3 times with chloroform (200mℓ), concentrated to evaporate hydrochloric acid, and diluted with water (55mℓ). The dilute solution was passed through a column packed with Dowex 50W-X2 ($H^+$ type, 50 ∿ 100 mesh, 100mℓ) and eluted with a series of water (300mℓ), 0.5N HCℓ, 0.8N HCℓ, 1.0N HCℓ, 1.2N HCℓ and 1.5N HCℓ (each 400mℓ) to fractionate 100mℓ portions of the eluate. Each resulting fraction was analyzed by thin-layer chromatography, after which the fractions containing the desired compound were combined together and concentrated. The resulting concentrate was diluted with water to make 30mℓ and again passed through a column packed with Dowex 50W-X2 ($H^+$ type, 50 ∿ 100 mesh, 30mℓ). The resulting effluent was further eluted with a series of 0.8N HCℓ, 0.9N HCℓ and1.0N HCℓ (each 150mℓ) to fractionate 30mℓ portions of the eluate. Each resulting fraction was analyzed in the same procedure as above. The fractions exhibiting a single spot were combined together, concentrated, and then lyophilized, yielding the dihydrochloride of Compound 23 in the form of a powder (301mg).

Optical rotation: $[\alpha]^{25}_D$ - 18.3° (c = 0.85, water)
Elemental analysis ($C_6H_{14}N_2O_3 2HC\ell$)
Calculated: C; 30.65, H; 6.86, N; 11.92, Cℓ; 30.16 (%)
Found :C; 30.30, H; 7.13, N; 12.07, Cℓ; 30.57 (%)

Example 20

Compound 16 (310mg) was dissolved in 2N HCℓ (31mℓ) and refluxed for 6 hours under heating. After cooling, the reaction liquid was washed 3 times with chloroform (40mℓ) and concentrated to evaporate hydrochloric acid. The resulting concentrate, after diluting with water (18mℓ), was passed through a column packed with Dowex 50W-X2 ($H^+$ type, 50 ∿ 100 mesh, 17mℓ) and eluted with a series of water (50mℓ), a 0.2% ammonia water solution (50mℓ), a 0.3% ammonia water solution (60mℓ) and a 0.4% ammonia water solution (60mℓ) to fractionate 17mℓ portions of the eluate. Each resulting fraction was analyzed by thin-layer chromatography. The fractions exhibiting a single spot were combined together, concentrated, and then lyophilized, yielding Compound 24 in the form of a powder (174mg). 38mg of the powder was dissolved in water (3.8mℓ), adsorbed to Dowex 50W-X2 ($H^+$ type, 50 ∿ 100 mesh, 5mℓ), and eluted with a series of water (15mℓ), 0.5N HCℓ, 0.8N HCℓ, 0.9N HCℓ and 1.0N HCℓ (each 20mℓ) to fractionate 5mℓ portions of the eluate. Each resulting fraction was analyzed by thin-layer chromatography. The fractions exhibiting a single spot were combined together, concentrated, and then lyophilized, yielding the dihydrochloride of Compound 24 (39mg).

Optical rotation: $[\alpha]^{23}_D$ - 2.7° (c = 0.58, water)
Elemental analysis ($C_7H_{16}N_2O_3 2HC\ell$)
Calculated: C; 34.02, H; 6.53, N; 11.34, Cℓ; 28.69 (%)
Found :C; 33.81, H; 7.81, N; 11.20, Cℓ; 29.52 (%)

Example 2I

The sodium salt of Compound 20 (280mg) was dissolved in a 0.03M phosphate buffer solution (pH 7.0, 140mℓ) and bacterial cells (14g) of Pseudomonasacidovorans were added, after which the mixture was shaken at 37°C for 20 hours. The reaction liquid was centrifuged; the resulting supernatant, after adjusting to pH 7.0, was concentrated. The resulting concentrate was subjected to column chromatography using Diaion HP-20 (50 ∿ 100 mesh, 140mℓ) as the packing and eluted with a series of water (900mℓ) and 5% methanol-water (500mℓ). Each resulting fraction was analyzed by high performance liquid chromatography [Mobile phase: 25% methanol/0.01M phosphoric acid solution (pH 3)]. The fractions exhibiting a single peak were combined together and concentrated. The resulting concentrate was then lyophilized, yielding Compound 25 in the form of a white powder (145mg).

Optical rotation: $[\alpha]^{23}_D$ + 10.3° (c = 0.48, water)
Elemental analysis ($C_{11}H_{22}N_2O_5$)
Calculated: C; 50.37, H; 8.45, N; 10.68 (%)
Found :C; 49.91, H; 8.54, N; 10.57 (%)

Example 22

Compound 21 (390mg) was suspended in a 0.03M phosphate buffer solution (pH 7.0, 200mℓ) and bacterial cells (40g) of Pseudomonasacidovorans were added, after which the mixture was shaken at 37°C for hours. The reaction liquid was centrifuged; the resulting supernatant, after adjusting to pH 7.0, was

concentrated. The resulting concentrate was subjected to column chromatography using Diaion HP-20 (50 ∿ 100 mesh, 140mℓ) as the packing and then eluted with a series of water (700mℓ) and 5% methanol-water (700mℓ) to fractionate 140mℓ portions of the eluate. Each resulting fraction was analyzed by high performance liquid chromatography [Mobile phase: 25% methanol/0.01M phosphate solution (pH 6.3)]. The fractions exhibiting a single peak were combined together and concentrated. The resulting concentrate was then lyophilized, yielding Compound 26 in the form of a white powder (94mg).

Optical rotation: $[\alpha]^{21.5}_D + 19.3°$ (c = 0.45, water)
Elemental analysis ($C_{12}H_{24}N_2O_5 0.5H_2O$)
Calculated: C; 50.51, H; 8.83, N; 9.82 (%)
Found :C; 50.53, H; 8.71, N; 9.82 (%)

Example 23

The hydrochloride of Compound 6 (9.5mg) was dissolved in 1N aqueous sodium hydroxide (0.95mℓ) and stirred at room temperature for 60 hours. After the completion of the reaction, the reaction liquid was diluted and analyzed by high performance liquid chromatography [Mobile phase: 60% methanol/0.0lM phosphoric acid solution (pH 3)]: it was found Compound 17 was produced in an amount of 3.8mg.

Example 24

The hydrochloride of Compound 10 (111mg) was dissolved in water (3.7mℓ) and sodium hydroxide (188mg) was added, after which the mixture was stirred at 60°C for about 16 hours. The reaction liquid, after adjusting to pH 6.7 with HCℓ, was diluted with water to make 13mℓ and subjected to high performance liquid chromatography [Mobile phase: 70% methanol/0.01M phosphoric acid solution (pH 3)] for analysis and quantitative determination: it was found that Compound 25 and Compound 20 were produced in amounts of 9mg and 32mg, respectively.

**Claims**

l. A compound representable by the formula

$$R^1-NH-\underset{R^2}{CH}-\underset{OH}{CH}-CH_2-\underset{R^3}{CH}-CH_2-COR^4$$

wherein $R^1$ is hydrogen, hexanoyl, or sorbyl, $R^2$ is hydrogen or methyl, $R^3$ is an amino which may optionally be protected, and $R^4$ is hydroxyl or 2-amidino-ethylamino, with the proviso that when both $R^1$ and $R^2$ are hydrogen and $R^3$is amino, $R^4$ is 2-amidino-ethylamino, or when $R^1$ is sorbyl and $R^4$ is 2-amidino-ethylamino, $R^3$ is a protected amino, or salts thereof.

2. A compound according to claim l, wherein $R'$ is sorbyl.
3. A compound according to claim l, wheren $R^2$ is methyl.
4. A compound according to claim l, wherein $R^3$ is a protected amino.
5. A compound according to claim l, wherein $R^4$ is 2-amidino-ethylamino.
6. A method for preparing a compound representable by the formula

$$R^1-NH-\underset{R^2}{CH}-\underset{OH}{CH}-CH_2-\underset{R^3}{CH}-CH_2-COOH$$

wherein $R^1$ is hydrogen, hexanoyl, or sorbyl, $R^2$ is hydrogen or methyl and $R^3$ is an amino which may optionally be protected, with the proviso that when both $R^1$ and $R^2$ are hydrogen, $R^3$ is a protected amino, or salts thereof, which comprises subjecting a compound representable by the formula

$$R^{1'}-NH-\underset{R^2}{CH}-\underset{OH}{CH}-CH_2-\underset{R^3}{CH}-CH_2-CONHCH_2CH_2\underset{\underset{NH}{\|}}{C}NH_2$$

wherein $R^{1'}$ is hexanoyl or sorbyl, $R^2$ and $R^3$ are the same meaning as defined above, or salts thereof, to hydrolysis.

7. A method according to claim 6, wherein the hydrolysis is conducted in the presence of an acid.
8. A method according to claim 7 wherein the acid is hydrochloric acid.
9. A method according to claim 6, wherein the hydrolysis is conducted in the presence of a base.
10. A method according to claim 9, wherein the base is sodium hydroxide.
11. A method for preparing a compound representable by the formula

$$R^{1''}-NH-\underset{R^2}{CH}-\underset{OH}{CH}-CH_2-\underset{R^3}{CH}-CH_2-COR^4$$

wherein $R^{1''}$ is hydrogen on hexanoyl, $R^2$ is hydrogen or methyl, $R^3$ is an amino which may optionally be protected, and $R^4$ is hydroxyl or 2-amidino-ethylamino, with the proviso that when both $R^{1''}$ and $R^2$ are hydrogen and $R^3$ is amino, $R^4$ is 2-amidino-ethylamino, or salts thereof, which comprises subjecting a compound representable by the formula

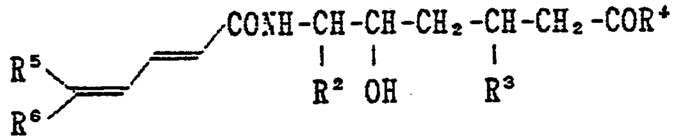

wherein $R^2$, $R^3$ and $R^4$ are the same meaning as defined above and $R^5$ and $R^6$ each are hydrogen or methyl, with the proviso that $R^5$ and $R^6$ are not simultaneously hydrogen or methyl, or salts thereof, to catalytic reduction, and when necessary, further to deacylation.

12. A method according to claim 11, wherein the catalytic reduction is conducted by use of palladium-carbon.
13. A method according to claim 11, wherein the deacylation is conducted by use of deacylase.
14. A method according to claim 13, wherein the deacylase is from Pseudomonasacidovorans IFOI3582.

## Patentansprüche

1. Verbindung, die durch die Formel

$$R^1-NH-\underset{R^2}{CH}-\underset{OH}{CH}-CH_2-\underset{R^3}{CH}-CH_2-COR^4$$

darstellbar ist worin $R^1$ Wasserstoff, Hexanoyl oder Sorbyl ist, $R^2$ Wasserstoff oder Methyl darstellt, $R^3$ eine gegebenenfalls geschützte Aminogruppe bedeutet und $R^4$ Hydroxyl oder 2-Amidinoäthylamino ist, mit der Maßgabe, daß wenn sowohl $R^1$ als auch $R^2$ für Wasserstoff stehen und $R^3$ Amino ist, $R^4$ 2-Amidinoäthylamino ist oder wenn $R^1$ für Sorbyl steht und $R^4$ 2-Amidinoäthylamino darstellt, $R^3$ eine geschützte Aminogruppe ist, oder Salze derselben.

2. Verbindung nach Anspruch, worin $R^1$ Sorbyl ist.
3. Verbindung nach Anspruch 1, worin $R^2$ für Methyl steht.
4. Verbindung nach Anspruch, worin $R^3$ geschütztes Amino darstellt.
5. Verbindung nach Anspruch 1, worin $R^4$ 2-Amidinoäthylamino bedeutet.
6. Verfahren zur Herstellung einer Verbindung, die durch die Formel

$$R^1-NH-\underset{R^2}{CH}-\underset{OH}{CH}-CH_2-\underset{R^3}{CH}-CH_2-COOH$$

darstellbar ist, worin $R^1$ Wasserstoff, Hexanoyl oder Sorbyl ist, $R^2$ für Wasserstoff oder Methyl steht und $R^3$ eine gegebenenfalls geschützte Aminogruppe darstellt, mit der Maßgabe, daß, wenn sowohl $R^1$ als auch $R^2$ Wasserstoff sind, $R^3$ für eine geschützte Aminogruppe steht, oder Salze derselben, umfassend das Hydrolysieren einer Verbindung, die durch die Formel

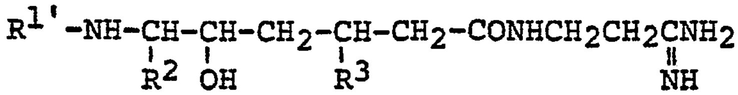

darstellbar ist, worin R¹, Hexanoyl oder Sorbyl ist und $R^2$ und $R^3$ die vorstehend angeführte Bedeutung besitzen, oder Salze derselben.

7. Verfahren nach Anspruch 6, worin die Hydrolyse in Gegenwart einer Säure durchgeführt wird.

8. Verfahren nach Anspruch 7, worin die Säure Salzsäure ist.

9. Verfahren nach Anspruch 6, worin die Hydrolyse in Gegenwart einer Base durchgeführt wird.

10. Verfahren nach Anspruch 9, worin die Base Natriumhydroxid ist.

11. Verfahren zur Herstellung einer Verbindung, die durch die Formel

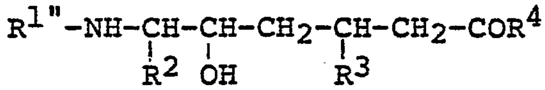

darstellbar ist, worin $R^{1''}$ Wasserstoff oder Hexanoyl ist, $R^2$ für Wasserstoff oder Methyl steht, $R^3$ eine gegebenenfalls geschützte Aminogruppe bedeutet und $R^4$ Hydroxyl oder 2-Amidinoäthylamino darstellt, mit der Maßgabe, daß, wenn sowohl $R^{1''}$ als auch $R^2$ Wasserstoff bedeuten und $R^3$ für Amino steht, $R^4$ 2-Amidinoäthylamino ist, oder Salze derselben, umfassend das Unterwerfen einer Verbindung, die durch die Formel

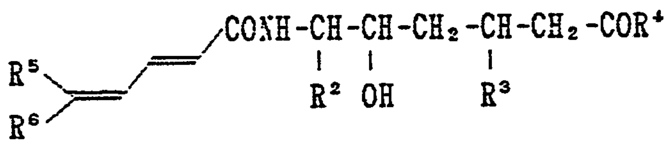

darstellbar ist, worin $R^2$, $R^3$ und $R^4$ die vorstehend angeführte Bedeutung besitzen und $R^5$ und $R^6$ jeweils für Wasserstoff oder Methyl stehen, mit der Maßgabe, daß $R^5$ und $R^6$ nicht gleichzeitig Wasserstoff oder Methyl sind, oder Salze derselben, der katalytischen Reduktion und nach Bedarf weiters der Desacylierung.

12. Verfahren nach Anspruch 11, worin die katalytische Reduktion unter Verwendung von Palladium auf Kohle durchgeführt wird.

13. Verfahren nach Anspruch 11, worin die Desacylierung unter Verwendung von Desacylase durchgeführt wird.

14. Verfahren nach Anspruch 13, worin die Desacylase von Pseudomonas acidovorans IFO13582 herrührt.

**Revendications**

1. Composé représenté par la formule

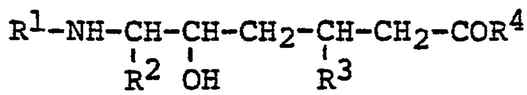

dans laquelle $R^1$ est un hydrogène, un groupe hexanoyle ou sorbyle, $R^2$ est un hydrogène ou un groupe méthyle, $R^3$ est un groupe amino qui peut être éventuellement protégé, et $R^4$ est un groupe hydroxyle ou 2-amidino-éthylamino, à condition que, lorsque $R^1$ et $R^2$ sont tous deux des atomes d'hydrogène et que $R^3$ est un groupe amino, $R^4$ soit un groupe 2-amidino-éthylamino, ou lorsque $R^1$ est un groupe sorbyle et $R^4$ est un groupe 2-amidino-éthylamino, $R^3$ soit un groupe amino protégé, ou ses sels.

2. Composé selon la revendication 1, dans lequel $R^1$ est un groupe sorbyle.

3. Composé selon la revendication 1, dans lequel $R^2$ est un groupe méthyle.

4. Composé selon la revendication 1, dans lequel $R^3$ est un groupe amino protégé.

5. Composé selon la revendication 1, dans lequel $R^4$ est un groupe 2-amidino-éthylamino.

6. Procédé de préparation d'un composé représenté par la formule

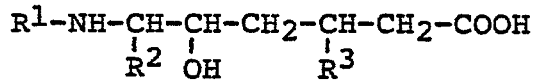

dans laquelle $R^1$ est un hydrogène, un groupe hexanoyle ou sorbyle, $R^2$ est un hydrogène ou un groupe méthyle et $R^3$ est un groupe amino qui peut être éventuellement protégé, à condition que, lorsque $R^1$ et $R^2$ sont tous deux des atomes d'hydrogène, $R^3$ soit un groupe amino protégé, ou de ses sels, qui comprend l'hydrolyse d'un composé représenté par la formule

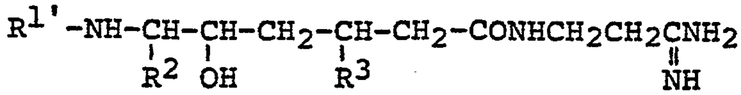

dans laquelle $R^1$, est un groupe hexanoyle ou sorbyle, $R^2$ et $R^3$ ont les mêmes significations que ci-dessus, ou de ses sels.

7. Procédé selon la revendication 6, dans lequel l'hydrolyse est réalisée en présence d'un acide.

8. Procédé selon la revendication 7, dans lequel l'acide est l'acide chlorhydrique.

9. Procédé selon la revendication 6, dans lequel l'hydrolyse est réalisée en présence d'une base.

10. Procédé selon la revendication 9, dans lequel la base est l'hydroxyde de sodium.

11. Procédé de préparation d'un composé représenté par la formule

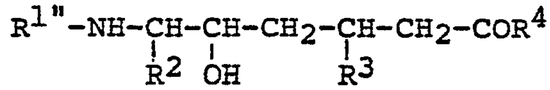

dans laquelle $R^{1''}$ est un hydrogène ou un groupe hexanoyle, $R^2$ est un hydrogène ou un groupe méthyle, $R^3$ est un groupe amino qui peut être éventuellement protégé et $R^4$ est un groupe hydroxyle ou 2-amidino-éthylamino, à condition que, lorsque $R^{1''}$ et $R^2$ sont tous deux des atomes d'hydrogène et que $R^3$ est un groupe amino, $R^4$ soit un groupe 2-amidino-éthylamino, ou de ses sels, qui comprend la réduction catalytique d'un composé représenté par la formule

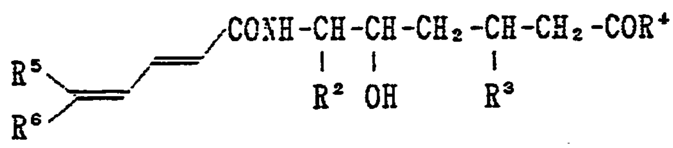

dans laquelle $R^2$, $R^3$ et $R^4$ ont la même signification que ci-dessus, et $R^5$ et $R^6$ sont chacun un hydrogène ou un groupe méthyle, à condition que $R^5$ et $R^6$ ne soient pas simultanément un hydrogène ou un groupe méthyle, ou de ses sels, et, le cas échéant, ensuite, une désacylation.

12. Procédé selon la revendication 11, dans lequel la réduction catalytique est réalisée au moyen de palladium-carbone.

13. Procédé selon la revendication 11, dans lequel la désacylation est réalisée au moyen d'une désacylase.

14. Procédé selon la revendication 13, dans lequel la désacylase provient de Pseudomonas acidovorans IFO13582.